# EUROPÄISCHE PATENTSCHRIFT

(11) **EP 2 445 862 B1**
(45) Veröffentlichungstag und Bekanntmachung des Hinweises auf die Patenterteilung: **05.09.2018**
(21) Anmeldenummer: 10725781.8
(22) Anmeldetag: 23.06.2010
(51) Int. Cl.: C07C 69/653, C07C 67/307, C07C 67/317, C07C 69/63

(54) **HERSTELLUNG VON SUBSTITUIERTEN 2-FLUORACRYLSÄUREDERIVATEN**
PREPARATION OF SUBSTITUTED 2-FLUOROACRYLIC ACID DERIVATIVES
PROCÉDÉ DE FABRICATION DE DÉRIVÉS SUBSTITUÉS DE L'ACIDE 2-FLUOROACRYLIQUE

(30) Priorität: 26.06.2009 DE 102009030681
(43) Veröffentlichungstag der Anmeldung: 02.05.2012
(73) Patentinhaber: Saltigo GmbH, 51369 Leverkusen (DE)
(72) Erfinder: KIRCHHOFF, Jan, 53844 Troisdorf (DE); KREIS, Michael, 51373 Leverkusen (DE)
(74) Vertreter: Matzke, Michael
(86) Internationale Anmeldenummer: PCT/EP2010/058870
(87) Internationale Veröffentlichungsnummer: WO 2010/149683

(56) Entgegenhaltungen:
- MUSLINKIN, A. A. ET AL: "Synthesis of some .alpha.-fluoroacryl esters" RUSSIAN JOURNAL OF APPLIED CHEMISTRY , 82(1), 116-122 CODEN: RJACEO; ISSN: 1070-4272, 1. Januar 2009 (2009-01-01), XP002600624
- ELKIK, ELIAS ET AL: "Fluoroalkylation of .beta.-dicarbonyl derivatives. Preparation of 4-fluorocyclohexane-1,3-diones" 1979, BULLETIN DE LA SOCIETE CHIMIQUE DE FRANCE , (1-2, PT. 2), 65-70 CODEN: BSCFAS; ISSN: 0037-8968 , XP002600625 Seite 65, Absatz A - Seite 66 Seite 68 - Seite 69, Spalte 1; Beispiele
- ELKIK, ELIAS ET AL: "Preparation of 3-amino-2-fluoropropionic and 2-fluoroacrylic esters" COMPTES RENDUS DES SEANCES DE L'ACADEMIE DES SCIENCES, SERIE C: SCIENCES CHIMIQUES , 282(24), 1129-31 CODEN: CHDCAQ; ISSN: 0567-6541, 28. Juni 1976 (1976-06-28), Seiten 1129-1131, XP008126927

## Beschreibung

Die vorliegende Erfindung betrifft ein Verfahren zur Herstellung von substituierten 2-Fluoracrylsäurederivaten.

Substituierte 2-Fluoracrylsäurederivate stellen Edukte zur Synthese von Polymeren dar. Diese können z.B. als Kunststoffe in optischen Lichtwellenleitern und als polymere Zusatzstoffe in Arzneimitteln eingesetzt werden.

Aus der Literatur sind verschiedene Verfahren zur Herstellung substituierter 2-Fluoracrylsäurederivate bekannt.

Aus Journal of Fluorine Chemistry, 55, 1991, S. 149 - 162 ist ein Verfahren zur Herstellung von substituierten 2-Fluoracrylsäurederivaten, insbesondere der 2-Fluoracrylsäureester, durch Hydrolyse von α-Hydroxymethyl-α-fluormalonsäureestern, anschließender Decarboxylierung und erneuter Veresterung bekannt. Das Verfahren weist den Nachteil auf, dass lediglich geringe Ausbeuten erhalten werden.

Ein weiteres Verfahren ist aus JP 2001172223 AA bekannt, in dem beschrieben wird, dass substituierte 2-Fluoracrylsäurederivate aus 2,2-Bromfluorpropionsäureestern hergestellt werden können. Nachteilig an diesem Verfahren ist, dass die Edukte schlecht verfügbar sind, das Verfahren daher ökonomisch nicht sinnvoll ist und lediglich geringe Ausbeuten erhalten werden.

In EP 0415214 A1 wird ein vierstufiges Verfahren zur Herstellung eines substituierten 2-Fluoracrylsäurederivates, nämlich des 2-Fluorarcrylsäureesters, ausgehend von 2,3-Dichlor-1-propen beschrieben. Weitere Verfahren zur Herstellung von substituierten 2-Fluoracrylsäurederivaten, ausgehend von 3-Hydroxy-2-fluorpropionaten durch Umsetzung mit Toluolsulfonsäurechlorid und Eliminierung des entstandenen Tosylates in Gegenwart von Kaliumphthalimid sind aus Journal of Fluorine Chemistry, 1993, 60, S. 149-162 und aus Coll. Czech. Chem. Commun., 1983, 48, S. 319-326 bekannt. Diese Verfahren haben gemeinsam, dass sie aus ökonomischen und sicherheitstechnischen Gründen in technischen Prozessen nicht einsetzbar sind.

Ein weiteres Verfahren zur Herstellung von substituierten 2-Fluoracrylsäurederivaten durch Umsetzung von 3-Hydroxy-2-fluorpropionaten mit Wasser entziehenden Mitteln ist aus Bull. Soc. Chem. Fr., 1975, S. 1633 - 1638 bekannt. Nachteilig an diesem Verfahren ist ebenfalls die geringe Reaktionsausbeute.

In Russian Journal of Applied Chemistry, 2009, 82(1) S. 116-122, wird ein Verfahren zur Herstellung von 2-Fluoracrylsäurederivaten offenbart, bei dem Verbindungen der Formel (II) in Gegenwart der Base Triethylamin umgesetzt werden. Aus Bull. Soc. Chim. Fr., 1979, 1-2, Pt. 2, S. 65-70, und Comptes Rendus des Seances de l'Academie des Sciences, Serie C: Sciences Chimiques, 1976, 282 (24), S. 1129-31, sind Verfahren zur Herstellung von 2-Fluoracrylsäurederivaten der allgemeinen Formel (I) durch die Umsetzung der Verbindungen der Formel (II) in Gegenwart von sekundären Aminen wie Dimethylamin als Basen bekannt.

Sämtlichen Verfahren ist gemeinsam, dass sie entweder aus sicherheitstechnischen und ökonomischen Gründen ungeeignet sind oder zu geringe Reaktionsausbeuten liefern. Es bestand daher weiterhin ein Bedürfnis nach einem Verfahren zur Herstellung von substituierten 2-Fluoracrylsäurederivaten, das die Nachteile des Standes der Technik überwindet und mit dem substituierte 2-Fluoracrylsäurederivate in effizienter Weise in technischen Prozessen hergestellt werden können.

Überraschend wurde ein Verfahren zur Herstellung von substituierten 2-Fluoracrylsäurederivaten gefunden, indem substituierte 3-Halogen-2-fluorpropionsäurederivate in Gegenwart von Basen zu substituierten 2-Fluorarylsäurederivaten in guten Ausbeuten und hohen Reinheiten umgesetzt werden.

Gegenstand der Erfindung ist daher ein Verfahren zur Herstellung von Verbindungen der Formel (I) 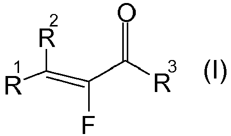
worin R¹ und R² gleich oder verschieden sind und unabhängig voneinander für Wasserstoff, C₁-C₁₅-Alkyl, C₆-C₂₄-Aryl, C₁-C₁₅-Alkoxy, C₂-C₁₀-Alkenyl, C₆-C₂₄-Aryloxy, C₇-C₁₅-Arylalkyl, C₁-C₁₅-Alkylthio, C₁-C₁₅-Mono- und Dialkylamino, C₆-C₂₄-Mono- und Diarylamino oder 5- bis 8-gliedriges gesättigtes oder ungesättigtes Heterozyklyl stehen, die gegebenenfalls weiter durch Reste ausgewählt aus der Gruppe C₁-C₁₅-Alkyl, C₂-C₁₀-Alkenyl, C₁-C₁₅-Alkoxy, C₆-C₂₄-Aryl, C₇-C₁₅-Arylalkyl, C₁-C₁₅-Alkylthio, Halogen, Hydroxy, Cyano, Nitro, Amino, Carboxy, C₁-C₁₅-Halogenalkyl, C₂-C₁₀-Halogenalkenyl, C₁-C₁₅-Halogenalkoxy, C₁-C₁₅-Halogenalkylthio und 5- bis 8- gliedriges gesättigtes oder ungesättigtes Heterozyklyl substituiert sein können, oder
R¹ und R² bilden zusammen einen carbozyklischen oder heterozyklischen, gesättigten oder ungesättigten 4- bis 8-gliedrigen Ring der gegebenenfalls weiter durch Reste ausgewählt aus der Gruppe C₁-C₁₅-Alkyl, C₂-C₁₀-Alkenyl, C₁-C₁₅-Alkoxy, C₆-C₂₄-Aryl, C₇-C₁₅-Arylalkyl, C₁-C₁₅-Alkylthio, Halogen, Hydroxy, Cyano, Nitro, Amino, Carboxy, C₁-C₁₅-Halogenalkyl, C₂-C₁₀-Halogenalkenyl, C₁-C₁₅-Halogenalkoxy, C₁-C₁₅-Halogenalkylthio und 5- bis 8- gliedriges gesättigtes oder ungesättigtes Heterozyklyl substituiert sein kann und
R³ steht für C₁-C₁₅-Alkoxy, C₁-C₁₅-Mono- und Dialkylamino, C₁-C₁₅-Alkylthio, C₆-C₂₄-Aryloxy, C₆-C₂₄-Mono- und Diarylamino und 5- bis 8- gliedriges gesättigtes oder ungesättigtes Heterozyklyl das über ein Heteroatom verknüpft ist, die gegebenenfalls weiter substituiert sein können durch Reste ausgewählt aus der Gruppe C₁-C₁₅-Alkyl, C₂-C₁₀-Alkenyl, C₁-C₁₅-Alkoxy, C₁-C₁₅-Alkylthio, C₆-C₂₄-Aryl, C₇-C₁₅-Arylalkyl, Halogen, Hydroxy, Cyano, Nitro, Amino, Carboxy, C₁-C₁₅-Halogenalkyl, C₂-C₁₆-Halogenalkenyl, C₁-C₁₅-Halogenalkoxy, C₁-C₁₅-Halogenalkylthio und 5- bis 8- gliedriges gesättigtes oder ungesättigtes Heterozyklyl,
bei dem Verbindungen der Formel (II) 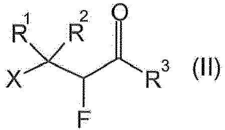
   worin R¹, R² und R³ die oben genannte Bedeutung besitzen
   und X für Cl, Br, I oder ein Pseudohalogen steht,
   in Gegenwart mindestens einer Base ausgewählt aus der Gruppe Erdalkali- oder Alkalimetallhydroxide, -carbonate, -hydrogenphosphate und -phosphate,
   und in Gegenwart mindestens eines Polymerisationsinhibitors
   zu Verbindungen der Formel (I) umgesetzt werden.

In einer bevorzugten Ausführungsform sind R¹ und R² unabhängig voneinander, können gleich oder verschieden sein und stellen bevorzugt Wasserstoff, C₁-C₆-Alkyl, C₆-C₂₄-Aryl, C₁-C₆-Alkoxy, C₂-C₆-Alkenyl, C₆-C₂₄-Aryloxy, C₇-C₁₀-Arylalkyl, C₁-C₆-Alkylthio, C₁-C₈-Mono- und Dialkylamino, C₆-C₂₄-Mono- und Diarylamino oder 5- bis 8- gliedriges gesättigtes oder ungesättigtes Heterozyklyl dar, die gegebenenfalls weiter durch Reste ausgewählt aus der Gruppe C₁-C₆-Alkyl, C₂-C₆-Alkenyl, C₁-C₆-Alkoxy, C₆-C₂₄-Aryl, C₇-C₁₅-Arylalkyl, C₁-C₆-Alkylthio, Amino, Halogen, Cyano, Carboxyl, Hydroxy, Nitro, C₁-C₆-Halogenalkyl, C₂-C₆-Halogenalkenyl, C₁-C₆-Halogenalkoxy, C₁-C₆-Halogenalkylthio und 5- bis 8- gliedriges gesättigtes und ungesättigtes Heterozyklyl substituiert sein können oder R¹ und R² bilden zusammen einen carbozyklischen oder heterozyklischen, gesättigten oder ungesättigten 4- bis 8-gliedrigen Ring, der gegebenenfalls weiter substituiert sein kann durch Reste ausgewählt aus der Gruppe Hydroxy, Cyano, Nitro, Amino, Carboxy, C₁-C₆-Alkyl oder C₆-C₂₄-Aryl. Besonders bevorzugt stellen R¹ oder/und R² C₁-C₆-Alkyl, C₁-C₆-Alkoxy, C₆-C₂₄-Alyl oder Wasserstoff dar. Ganz besonders bevorzugt stellen R¹ oder/und R² Methyl, Ethyl, Propyl, i-Propyl, n-, i-, s- oder t-Butyl, n-Pentyl, Phenyl oder Wasserstoff dar.

R³ stellt bevorzugt C₁-C₆-Alkoxy, C₁-C₈-Mono- und Dialkylamino, C₁-C₆-Alkylthio, C₆-C₂₄-Aryloxy, C₆-C₂₄-Mono- und Diarylamino oder 5- bis 8- gliedriges gesättigtes oder ungesättigtes Heterozyklyl das über ein Stickstoffatom verknüpft ist, dar, die gegebenenfalls weiter durch Reste ausgewählt aus der Gruppe C₁-C₆-Alkyl, C₂-C₆-Alkenyl, C₁-C₆-Alkoxy, C₆-C₂₄-Aryl, C₇-C₁₅-Arylalkyl, C₁-C₆-Alkylthio, Amino, Halogen, Cyano, Carboxyl, Hydroxy, Nitro, C₁-C₆-Halogenalkyl, C₂-C₆-Halogenalkenyl, C₁-C₆-Halogenalkoxy oder C₁-C₆-Halogenalkylthio substituiert sein können. Besonders bevorzugt steht R³ für Methoxy, Ethoxy, n-Propoxy, Isopropoxy, t-Butoxy, n-Pentoxy, n-Hexoxy, Cyclopropoxy, Cyclobutoxy, Cyclopentoxy, Cyclohexoxy, Cycloheptoxy, Ethylamino, Diethylamino, Methylamino, Dimethylamino, Butylamino, Dibutylamino, Propylamino, Dipropylamino, Benzylamino, Phenoxy, Pyrrolidinyl, Piperazinyl oder Pyridazinyl. Ganz besonders bevorzugt sind Methoxy, Ethoxy, Propoxy, Methylamin, Ethylamin, Pyrrolidinyl, Piperazinyl oder Pyridazinyl.

X steht bevorzugt für Cl oder Br. Besonders bevorzugt steht X für Chlor.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Alkyl bzw. Alkoxy bzw. Alkylthio steht im Rahmen der Erfindung für einen geradkettigen, zyklischen oder verzweigten Alkyl- bzw. Alkoxy- bzw. Alkylthio- Rest mit 1 bis 15 (C₁-C₁₅), bevorzugt 1 bis 12 (C₁-C₁₂) und besonders bevorzugt 1 bis 6 (C₁-C₆). Beispielhaft steht Alkyl für Methyl, Ethyl, n-Propyl, Isopropyl, n-, i-, s- oder t-Butyl, Cyclopropyl, Cyclobutyl, Cyclopentyl, Cyclohexyl, Cycloheptyl, n-Pentyl, 1-Methylbutyl, 2-Methylbutyl, 3-Methylbutyl, neo-Pentyl, 1-Ethylpropyl, n-Hexyl, n-Heptyl, n-Octyl, n-Decyl und n-Dodecyl. Vorzugsweise steht Alkyl für Methyl, Ethyl, n-Propyl, Isopropyl, n-, i-, s- oder t-Butyl, Cyclopropyl, Cyclobutyl, Cyclopentyl, n-Hexyl, Cyclohexyl, n-Pentyl, 1-Methylbutyl, 2-Methylbutyl, 3-Methylbutyl, neo-Pentyl, 1-Ethylpropyl, cyclo-Hexyl. Besonders bevorzugt steht Alkyl für Methyl, Ethyl, n-Propyl, Isopropyl, n-, i-, s- oder t-Butyl, n-Pentyl und n-Hexyl. Beispielhaft und vorzugsweise steht Alkoxy Methoxy, Ethoxy, n-Propoxy, Isopropoxy, n-,i-,s- oder t-Butoxy, n-Pentoxy, n-Hexoxy, Cyclopropoxy, Cyclobutoxy, Cyclopentoxy, Cyclohexoxy oder Cycloheptoxy. Beispielhaft und vorzugsweise steht Alkylthio für Methanthiol, Ethanthiol, n-Propanthiol, Isopropanthiol, n-, i-, s- oder t-Butanthio, n-Pentanthio, 1-Methylbutanthio, 2-Methylbutanthio, 3-Methylbutanthio, neo-Pentanthio, 1-Ethylpropanthio und n-Hexanthio.

Alkenyl steht im Rahmen der Erfindung für einen geradkettigen, zyklischen oder verzweigten Alkenylrest mit 2 bis 10 (C₂-C₁₀), bevorzugt mit 2 bis 6 (C₂-C₆) Kohlenstoffatomen. Beispielhaft und vorzugsweise steht Alkenyl für Vinyl, Allyl, Isopropenyl und n-But-2-en-1-yl.

Aryl steht im Rahmen der Erfindung für einen aromatischen Rest mit 6 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 24 Gerüstkohlenstoffatomen. Gleiches gilt für den aromatischen Teil eines Arylalkyl-Restes. Weiterhin können die carbozyklischen aromatischen oder heteroaromatischen Reste mit bis zu fünf gleichen oder verschiedenen Substituenten pro Zyklus substituiert sein, ausgewählt aus der Gruppe C₁-C₆-Alkyl, C₂-C₆-Alkenyl, C₁-C₆-Alkoxy, Amino, C₇-C₁₅-Arylalkyl, Carboxyl, C₁-C₈-Mono- und Dialkylamino, Halogen, Nitro, Cyano, Carboxy, C₁-C₆-Halogenalkyl, C₁-C₆-Halogenalkenyl, C₁-C₆-Halogenalkoxy, C₁-C₆-Halogenalkylthio oder Hydroxy.

Bevorzugte Beispiele für C₆-C₂₄-Aryl sind Phenyl, o-, p-, m-Tolyl, Naphthyl, Phenanthrenyl, Anthracenyl oder Fluorenyl, Beispiele für heteroaromatisches C₆-C₂₄-Aryl in denen ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind Pyridyl, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Benzofuranyl oder Dibenzofuranyl.

Arylalkyl bedeutet jeweils unabhängig voneinander einen geradkettigen, zyklischen oder verzweigten Alkyl-Rest nach vorstehender Definition, der einfach, mehrfach oder vollständig durch Aryl-Reste gemäß vorstehender Definition substituiert sein kann. Ein Beispiel für Arylalkyl ist Benzyl. Bevorzugt sind Arylalkyle mit 7 bis 15 (C₇-C₁₅) Kohlenstoffatome, besonders bevorzugt sind Arylalkyle mit 7 bis 10 (C₇-C₁₀) Kohlenstoffatomen.

Aryloxy steht im Rahmen der Erfindung für einen C₆-C₂₄-Arylrest der vorstehenden Definition der über ein Sauerstoffatom verknüpft ist. Beispielsweise kann der Arylrest weiter substituiert sein durch Reste ausgewählt aus der Gruppe C₁-C₆-Alkyl, C₂-C₆-Alkenyl, C₁-C₆-Alkoxy, Amino, C₇-C₁₅-Arylalkyl, Carboxyl, C₁-C₈-Mono- und Dialkylamino, Halogen, Nitro, Cyano, Carboxy, C₁-C₆-Halogenalkyl, C₁-C₆-Halogenalkenyl, C₁-C₆-Halogenalkoxy, C₁-C₆-Halogenalkylthio oder Hydroxy. Beispielsweise und vorzugsweise ist Aryloxy Phenoxy.

Mono- und Diarylamino steht im Rahmen der Erfindung für eine Amino-Gruppe die mit einem oder zwei gleichen oder verschiedenen, C₆-C₂₄-Arylrest der vorstehenden Definition verbunden ist und über das Stickstoffatom verknüpft ist. Beispielsweise kann der Arylrest weiter substituiert sein durch Reste ausgewählt aus der Gruppe C₁-C₆-Alkyl, C₂-C₆-Alkenyl, C₁-C₆-Alkoxy, Amino, C₇-C₁₅-Arylalkyl, Carboxyl, C₁-C₈-Mono- und Dialkylamino, Halogen, Nitro, Cyano, Carboxy, C₁-C₆-Halogenalkyl, C₁-C₆-Halogenalkenyl, C₁-C₆-Halogenalkoxy, C₁-C₆-Halogenalkylthio oder Hydroxy.

5- bis 8- gliedriges gesättigtes oder ungesättigtes Heterozyklyl steht im Rahmen der Erfindung vorzugsweise für einen gesättigten oder ungesättigten Heterozyklus-Rest, mit bis zu 3 gleichen oder verschiedenen Heteroatomen aus der Reihe S, N und/oder O, der über ein Ringkohlenstoffatom, Ringstickstoffatom, Ringsauerstoffatom oder Ringschwefelatom verknüpft ist. Beispielsweise kann das 5- bis 8- gliedrige gesättigte oder ungesättigte Heterozyklyl weiter substituiert sein durch Reste ausgewählt aus der Gruppe C₁-C₆-Alkyl, C₂-C₆-Alkenyl, C₁-C₆-Alkoxy, Amino, C₇-C₁₅-Arylalkyl, Carboxyl, C₁-C₈-Mono- und Dialkylamino, Halogen, Nitro, Cyano, Carboxy, C₁-C₆-Halogenalkyl, C₁-C₆-Halogenalkenyl, C₁-C₆-Halogenalkoxy, C₁-C₆-Halogenalkylthio oder Hydroxy. Bevorzugt ist ein 5- bis 8-gliedriges gesättigtes Heterozyklyl mit bis zu 2 gleichen oder verschiedenen Heteroatomen aus der Reihe S, N und/oder O. Beispielhaft und vorzugsweise seien genannt Azepanyl, Pyrrolidinyl, Piperidinyl, Piperazinyl, Morpholinyl oder Tetrahydrofuryl.

Monoalkylamino bzw. Dialkylamino steht im Rahmen der Erfindung für eine Amino-Gruppe die mit einem oder zwei gleichen oder verschiedenen, zyklischen oder nicht zyklischen, geradkettigen oder verzweigten Alkylsubstituenten verbunden ist, der bzw. die vorzugsweise jeweils 1 bis 15, Kohlenstoffatome aufweist. Besonders bevorzugt ist die Aminogruppe mit einem oder zwei gleichen oder verschiedenen, zyklischen oder nicht zyklischen, geradkettigen oder verzweigten Alkylsubstituenten verbunden, der bzw. die vorzugsweise 1 bis 12, Kohlenstoffatome aufweist, ganz besonders bevorzugt 1 bis 8 Kohlenstoffatome aufweist. Beispielsweise kann der Alkylrest weiter substituiert sein durch Reste ausgewählt aus der Gruppe C₁-C₆-Alkyl, C₂-C₆-Alkenyl, C₁-C₆-Alkoxy, Amino, C₇-C₁₅-Arylalkyl, Carboxyl, C₁-C₈-Mono- und Dialkylamino, Halogen, Nitro, Cyano, Carboxy, C₁-C₆-Halogenalkyl, C₁-C₆-Halogenalkenyl, C₁-C₆-Halogenalkoxy, C₁-C₆-Halogenalkylthio oder Hydroxy.

Beispielhaft und vorzugsweise steht Monoalkylamino für Methylamino, Ethylamino, n-Propylamino, Isopropylamino, t-Butylamino, n-Pentylamino und n-Hexylamino.

Beispielhaft und vorzugsweise steht Dialkylamino für N,N-Dimethylamino, N,N-Diethylamino, N-Ethyl-N-methylamino, N-Methyl-N-n-propylamino, N-Isopropyl-N-n-propylamino, N-t-Butyl-N-methylamino, N-Ethyl-N-n-pentylamino und N-n-Hexyl-N-methylamino.

Halogenalkyl bzw. Halogenalkenyl bzw. Halogenalkoxy steht im Rahmen der Erfindung für einen geradkettigen, zyklischen, oder verzweigten Alkyl-, bzw. Alkenyl-, bzw. Alkoxy-Rest nach vorstehender Definition, der einfach, mehrfach oder vollständig mit Halogenatomen substituiert ist.

Beispielhaft und vorzugsweise steht C₁-C₁₅-Halogenalkyl für Dichlormethyl, Difluormethyl, Fluormethyl, Trifluormethyl, Trichlormethyl, 2,2,2-Trifluorethyl, Trifluormethyl, Pentafluorethyl, Heptafluorpropyl, cylco-Nonafluorpentyl, cyclo-Nonachlorpentyl, Heptafluorisopropyl und Nonafluorbutyl.

Beispielhaft und vorzugsweise steht C₂-C₆-Halogenalkenyl für Chlorehtylen, Dichlorethylen oder Trifluorethylen.

Beispielhaft und vorzugsweise steht C₁-C₆-Halogenalkoxy für Difluormethoxy, Fluorethoxy, Fluormethoxy, Trifluormethoxy, Trichlormethoxy und 2,2,2-Trifluorethoxy.

Halogenalkylthio bedeutet im Rahmen der Erfindung einen geradkettigen, zyklischen oder verzweigten Rest mit 1 bis 15 Kohlenstoffatomen, bevorzugt mit 1 bis 6 (C₁-C₆) Kohlenstoffatomen, der einfach, mehrfach oder vollständig mit Halogenatomen substituiert ist. Beispielhaft und vorzugsweise steht C₁-C₁₅-Halogenalkylthio für Chlorethylthio, Chlorbutylthio, Chlorhexylthio, Chlorpentylthio, Chlordodecylthio, Dichlorethylthio, Fluorethylthio, Trifluormethylthio und 2,2,2-Trifluorethylthio.

Halogen steht für Fluor, Chlor, Brom oder Iod. Pseudohalogen kann beispielsweise und vorzugsweise für Cyanid, Cyanat oder Thiocyanat stehen.

Basen im Sinne der Erfindung stellen z.B. Erdalkali- oder Alkalimetallhydroxide, -carbonate, - hydrogenphosphate, -phosphate dar. Bevorzugt ist der Einsatz von Kaliumphosphat, Dinatriumhydrogenphosphat, Natriumphosphat, Kaliumcarbonat, Natriumcarbonat, Natriumhydroxid, Kaliumhydroxid oder Mischungen dieser Basen. Besonders bevorzugt werden als Base Natriumcarbonat, Kaliumcarbonat, Natriumphosphat oder Kaliumphosphat eingesetzt. Das erfindungsgemäße Verfahren kann beispielsweise in Gegenwart oder in Abwesenheit eines Lösungsmittels durchgeführt werden. Bevorzugt wird das erfindungsgemäße Verfahren in Gegenwart eines Lösungsmittels durchgeführt.
Als Lösungsmittel im erfindungsgemäßen Verfahren können beispielsweise und vorzugsweise aprotisch, polare Lösungsmittel wie Sulfoxide wie z.B. Dimethylsulfoxid, wie Ether, wie z.B. Polyethylenglycolen, Ethylenglycoldimethylether, Ethylenglycoldiethylether, Ethylenglycoldibutylether, Diethylenglykoldimethyether, Tetraethylenglycoldimethylether, Dioxan, Tetrahydrofuran oder 1,2-Dimethoxyethan, wie Sulfone, wie z.B. Sulfolan, Tetramethylsulfon, wie amidische Lösungsmittel, wie z.B. Dimethylformamid, N-Methyl-pyrrolidon, N-Methylcaprolactam oder Dimethylacetamid, wie Ketone, wie z.B. Dipropylketon oder Methyl-tert.-butylketon oder Nitrile, wie z.B. Benzonitril oder Benzylnitril oder halogenierte Aromaten wie z.B. Chlorbenzol, ortho-Dichlorbenzol oder Mischungen solcher Lösungsmittel eingesetzt werden. Bevorzugt werden als aprotische, polare Lösungsmittel Polyethylenglycolen, Ethylenglycoldimethylether, Ethylenglycoldiethylether, Ethylenglycoldibutylether, Diethylenglykoldimethyether, Tetraethylenglycoldimethylether, Dimethylformamid, Dimethylsulfoxid, Sulfolan, ortho-Dichlorbenzol oder N-Methyl-pyrrolidon oder Mischungen solcher Lösungsmittel eingesetzt. Besonders bevorzugt wird das erfindungsgemäße Verfahren in Gegenwart eines hochsiedenden Lösungsmittels durchgeführt. Im Rahmen der Erfindung gilt ein Lösungsmittel dann als hochsiedend, wenn der Siedepunkt des Lösungsmittels bei einem Druck von 1 bar ≥ 140 °C ist. Ganz besonders bevorzugt werden als hochsiedende, aprotische, polare Lösungsmittel Ethylenglycoldibutylether, Diethylenglykoldimethyether, Tetraethylenglycoldimethylether, Dimethylformamid, Dimethylsulfoxid, Sulfolan, ortho-Dichlorbenzol oder N-Methyl-pyrrolidon oder Mischungen solcher Lösungsmittel eingesetzt.

Als Polymerisationsinhibitor können beispielsweise und vorzugsweise freie Nitroxylradikale, wie beispielsweise 2,2,6,6-Tetramethylpiperidin-N-oxyl, Schwefel, p-Benzochinon, 4-tert.-Butylbrenzcatechin, Phenothiazin Di-tert-butylhydroxyltoluol (BHT) oder stcrisch gehinderte Phenole oder Mischungen von diesen Polymerisationsinhibitoren eingesetzt werden. Bevorzugt wird Di-tert-butylhydroxyltoluol (BHT) eingesetzt.

Das erfindungsgemäße Verfahren wird vorzugsweise bei einer Temperatur von 50 °C bis 200 °C, besonders bevorzugt bei 70 bis 180 °C, ganz besonders bevorzugt bei 130 °C bis 170 °C durchgeführt.

Das molare Verhältnis des Polymerisationsinhibitors und der eingesetzten Verbindungen der Formel (II) liegt beispielsweise zwischen 1*10⁻⁶ und 1*10⁻¹, bevorzugt zwischen 5*10⁻⁶ und 5*10⁻³ , besonders bevorzugt zwischen 1*10⁻⁵ und 1*10⁻³.

Das molare Verhältnis der eingesetzten zu Verbindungen der Formel (II) und der eingesetzten Base liegt beispielsweise zwischen 0,1 und 10, bevorzugt zwischen 0,3 und 2, besonders bevorzugt zwischen 0,5 und 1,5.

Im erfindungsgemäßen Verfahren werden beispielsweise Polymerisationsinhibitor, Lösungsmittel und Base zunächst vorgelegt. Beispielsweise kann die Mischung dann auf die Reaktionstemperatur erhitzt werden und ein Vakuum angelegt werden. Beispielsweise kann dann mit der Zugabe der Verbindungen der Formel (II) begonnen werden. Ebenso könnte beispielsweise auch zunächst nur die Base und das Lösungsmittel vorgelegt werden und der Polymerisationinhibitor mit den Verbindungen der Formel (II) vermischt werden und beispielsweise erst dann zugegeben werden. Die Zugabe der Reaktionskomponenten und Mischungen erfolgt z.B. dosiert. Die Verbindungen der Formel (I) können beispielsweise im erfindungsgemäßen Verfahren kontinuierlich, z.B. durch Destillation, abgetrennt werden.

Bevorzugt wird das erfindungsgemäße Verfahren so durchgeführt, dass der Polymerisationsinhibitor, das Lösungsmittel und die Base zunächst vorgelegt werden und die Mischung auf Reaktionstemperatur erwärmt wird. Bevorzugt wird dann ein Vakuum angelegt und die Verbindungen der Formel (II) zugegeben. Vorzugsweise werden während der Reaktion die entstehenden Verbindungen der Formel (I) abgetrennt. Bevorzugt geschieht dies durch Destillation. Viele der im erfindungsgemäßen Verfahren eingesetzten Verbindungen sind handelsüblich oder können nach analogen, aus dem Stand der Technik bekannten Verfahren hergestellt werden, die dem Fachmann bekannt sind.

Bevorzugt ist es allerdings die Verbindungen der Formel (II) aus Verbindungen der Formel (III) herzustellen, in dem die Verbindungen der Formel (III) 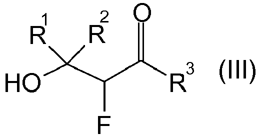
worin R¹ , R² und R³ die bei den Verbindungen der Formel (I) genannte Bedeutung aufweisen,
gegebenenfalls in Gegenwart von mindestens einem Lösungsmittel und
gegebenenfalls in Gegenwart mindestens einer Base
mit Halogenierungsmitteln zu Verbindungen der Formel (II) umgesetzt werden.

Als Halogenierungsmittel zur Herstellung der Verbindungen der Formel (II) können beispielsweise und vorzugsweise Thionylchlorid, Thionylbromid, Phosphortrichlorid, Phosphortribromid, Sulfurylchlorid, Sulfurylbromid, HX mit X = Cl, Br oder F oder Mischungen dieser Verbindungen eingesetzt werden. Besonders bevorzugt wird Thionylchlorid eingesetzt.

Bevorzugt wird das Verfahren zur Herstellung der Verbindungen der Formel (II) in Gegenwart von mindestens einem inerten Lösungsmittel durchgeführt. Bevorzugte Lösungsmittel im Verfahren zur Herstellung der Verbindungen der Formel (II) sind inerte, unpolare, aliphatische oder aromatische Lösungsmittel wie beispielsweise Benzol, Toluol, Xylol, verschiedene Petrolether, Hexan, Cyclohexan, gegebenenfalls halogenierte Kohlenwasserstoffe, wie z.B. Tetrachlorkohlenstoff, oder Gemische solcher organischer Lösungsmittel. Besonders bevorzugt sind Benzol, Hexan, Petrolether, Toluol, p-Xylol und Xylol Isomerengemisch oder Mischungen solcher Lösungsmittel.

Zudem wird die Herstellung der Verbindungen der Formel (II) aus den Verbindungen der Formel (III) bevorzugt in Gegenwart einer Base durchgeführt. Als Basen werden in diesem Fall vorzugsweise Erdalkali- oder Alkalimetallhydroxide, -alkoholate, -carbonate, -hydrogenphosphate, -phosphate wie beispielsweise Kalium-tert.-butylat, Natriumhydroxid, Kaliumhydroxid, Natriumcarbonat, Kaliumcarbonat, Natriumphosphat, Kaliumphosphat, Natriumhydrogenphosphat, Kaliumhydrogenphosphat, sowie tertiäre Amine, wie Trimethylamin, Triethylamin, Tributylamin, Di-isopropyl-ethylamin, N,N-Dimethylanilin, Piperidin, Pyridin, N-Methylpiperidin, N,N-Dimethylaminopyridin sowie 1,4-Diazabicyclo[2.2.2]octan (DABCO), 1,5-Diazabicyclo[4.3.0]non-5-en (DBN), 1,8-Diazabicyclo[5.4.0]undec-7-en (DBU), 1,1,3,3-Tetramethylguanidin (TMG), 7-Methyl-1,5,7-triazabicyclo[4.4.0]dec-5-en (MTBD) und 2,8,9-Triisopropyl-2,5,8,9-tetraaza-1-phosphabicyclo[3.3.3]undecan (TTPU) oder Mischungen dieser Basen eingesetzt. Besonders bevorzugt ist der Einsatz von bi- oder trizyklischen Basen, Pyridin, N-Methylpiperidin, N,N-Dimethylaminopyridin oder Piperidin oder Mischungen dieser Basen.

Das Verfahren zur Herstellung der Verbindungen der Formel (II) aus Verbindungen der Formel (III) wird vorzugsweise bei einer Temperatur von 0°C bis 25 °C, besonders bevorzugt bei 0°C bis 5 °C durchgeführt.

Das molare Verhältnis der Halogenierungsmittel zu den Verbindungen der Formel (III) liegt beispielsweise zwischen 0,8 und 1,5 und bevorzugt zwischen 1,0 und 1,5.

Vorzugsweise wird die Herstellung der Verbindungen der Formel (II) aus den Verbindungen der Formel (III) im Wesentlichen wasserfrei durchgeführt. Im Wesentlichen wasserfrei bedeutet, dass der Wassergehalt bezogen auf die Menge der eingesetzten Reaktionsmischung vorzugsweise zwischen 0.0001 Gew. % und 1,0 Gew.% liegt.

Das Verfahren zur Herstellung der Verbindungen der Formel (II) aus den Verbindungen der Formel (III) wird beispielsweise so durchgeführt, dass zunächst die Base und das Lösungsmittel vorgelegt werden. Dieses Gemisch kann beispielsweise zunächst auf 30 bis 100 °C erwärmt werden. Bevorzugt wird das Gemisch auf 60 bis 90 °C erwärmt. Durch Zugabe bei einer Temperatur von 30°C bis 100 °C kann die Bildung von störenden Nebenkomponenten deutlich reduziert werden. Beispielsweise werden in einem separaten Behälter das Halogenierungsmittel und die Verbindungen der Formel (III) vermischt. Das Gemisch aus dem Halogenierungsmittel und der Verbindungen der Formel (III) kann dann zu dem Gemisch aus Base und Lösungsmittel zugegeben werden. Beispielsweise können aber ebenfalls zunächst die Halogenierungsmittel und die Verbindungen der Formel (III) vermischt und vorgelegt werden. Beispielsweise kann dann die Mischung aus Base und Lösungsmittel zunächst erwärmt werden und dann beispielsweise bei einer Temperatur von 30 °C bis 100 °C zugegeben werden. Beispielsweise erfolgt die Zugabe der Reaktionskomponenten und Mischungen dosiert.

Bevorzugt wird das Verfahren zur Herstellung der Verbindungen der Formel (II) aus den Verbindungen der Formel (III) zunächst so durchgeführt, dass die Base und das Lösungsmittel vorgelegt werden. Bevorzugt wird diese Mischung dann auf 60 °C bis 90 °C erwärmt. Bevorzugt wird in einem anderen Behälter das Halogenierungsmittel vorgelegt und die Verbindungen der Formel (III) hinzugegeben. Diese Mischung wird bevorzugt dosiert zu der Mischung aus Base und Lösungsmittel hinzugegeben.

Die Verbindungen der Formel (III) sind handelsüblich oder können nach analogen, aus dem Stand der Technik bekannten Verfahren hergestellt werden, die dem Fachmann bekannt sind.

Mit Hilfe des Verfahrens zur Herstellung der Verbindungen der Formel (II) aus den Verbindungen der Formel (III) lässt sich die Bildung von störenden Nebenprodukten vermindern. Zudem wird mit dem Verfahren auch bei Einsatz geringer Mengen von Halogenierungsmitteln und bei niedrigen Temperaturen Verbindungen der Formel (III) in guten Ausbeuten hergestellt.

Die Aufreinigung der Verbindungen der Formel (II) sowie der Verbindungen der Formel (I) kann nach dem Fachmann bekannten Verfahren, beispielsweise durch Extraktion mit Lösungsmitteln, Destillation oder Kristallisation, erfolgen.

Durch das erfindungsgemäße Verfahren können die Verbindungen der Formel (I) in hohen Ausbeuten und in hoher Reinheit auf technisch einfache Weise hergestellt werden. Das erfindungsgemäße Verfahren erfordert nicht die Handhabung von Chemikalien, die wegen ihres Gefahrenpotenzials besonderen Aufwand erfordern und es ist ohne Probleme auch in größerem Maßstab durchführbar. Es ist vor allem überraschend, dass das erfindungsgemäße Verfahren die Verbindungen der Formel (I) in hohen Ausbeuten und in hoher Reinheit liefert.

Die erfindungsgemäß hergestellten Verbindungen der Formel (I) eignen sich insbesondere zur Herstellung von Kunststoffen und polymerer Zusatzstoffe in Arzneimitteln.

Die nachstehenden Beispiele dienen der Erläuterung der Erfindung, ohne dabei auf diese beschränkt zu sein.

### Beispiele

### 1. Herstellung von 3-Chlor-2-Fluorpropionsäuremethylester

743 g Thionylchlorid (6,3 mol) werden in einem Sulfierbecher vorgelegt und auf 5°C gekühlt. Bei 5±5°C werden im Maße der Gasentwicklung 748 g (98%ig, 6,00 mol) 3-Hydroxy-2-Fluorpropionsäuremethylester zudosiert. Nach vollständiger Dosierung wird bei 0°C bis 30°C nachgerührt. In einem zweiten Sulfierbecher werden 24 g (0,30 mol) Pyridin und 332 g Toluol vorgelegt, auf 80°C erwärmt und hierzu im Maße der Gasentwicklung das Chlorierungsgemisch aus Sulfierbecher 1 dosiert. Nach vollständiger Dosierung wird bei 80°C nachgerührt. Anschließend wird unter vermindertem Druck (200 - 500 mbar) über eine Füllkörperkolonne zuerst Toluol abdestilliert, gefolgt von der fraktionierenden Destillation des Produktes bei 20 mbar (Siedepunkt: 70°C). Es werden 742 g (5,28 mol, 88% Ausbeute) Produkt als farblose Flüssigkeit erhalten.

### 2. Herstellung von 2-Fluoracrylsäuremethylester

In einem Sulfierbecher werden 4 g (0,02 mol) Di-tert-butylhydroxytoluol (BHT), 500 g N-Methylpyrrolidin (NMP) und 360 g (2,2 mol) Natriumphosphat, tribasisch vorgelegt und auf 150°C erhitzt. In den Vorlagekolben der Destillationsbrücke werden 51 mg (2,2 *10⁻⁴ mol) Di-*tert-*butylhydroxytoluol (BHT) vorgelegt. Es wird ein Vakuum von 300 mbar angelegt und mit der Dosierung von insgesamt 287 g (98%ig, 2,0 mol) 3-Chlor-2-Fluorpropionsäuremethylester begonnen. Die Dosierung wird in dem Maße fortgesetzt in dem der 2-Fluoracrylsäuremethylester überdestilliert. Nachdem kein Destillatstrom mehr übergeht wird das Vakuum auf 150 mbar erniedrigt. Geht auch hier kein Produkt mehr über wird der Druck durch Zufuhr von Stickstoff erhöht. Der Destillationssumpf wird abgekühlt und über das Bodenventil abgelassen. Das Produkt wird nach Destillation als farblose Flüssigkeit erhalten (205 g, 95%ig, 1,87 mol, 93,5% Ausbeute).

### 3. Herstellung von 3-Chlor-2-Fluorpropionsäureethylester

In einem trockenen 50 ml Rundkolben unter Stickstoff-Atmosphäre wurden 8,99 g (0,08 mol) Thionylchlorid bei 20-25 °C vorgelegt. Anschließend wurde 3-Hydroxy-2-Fluor-propionsäureethylester (10,0 g, 0,07 mol) innerhalb von 1 h zudosiert und anschließend bei Raumtemperatur noch 2 h gerührt. In einem zweiten Rundkolben mit Anschützaufsatz wurden 3,98 g Toluol (0,04 mol) zusammen mit 0,286 g Pyridin (5 mol%) vorgelegt und auf 80 °C erwärmt. Zur vorgeheizten Lösung wurde die Mischung aus dem 1. Rundkolben innerhalb von 1 h bei 75-85 °C zugetropft. Lösungsmittel wurden unter vermindertem Druck abdestilliert und anschließend der Rückstand bei 120 °C und 10 mbar feindestilliert. Man erhielt 4,1 g Destillat als farblose Flüssigkeit.
1H-NMR (400 mHz, CDCl3): 5,67 (dd; J = 44 Hz, 3 Hz; CHH); 5,31 (dd; J = 13 Hz, 3 Hz; CHH); 4,30 (q, J = 7 Hz, CH2), 1,35 (t, J = 7 Hz, CH3) ppm.

### 4. Herstellung von 2-Fluoracrylsäureethylester

In einem 3-Halskolben wurden 10 ml (0,1 mol) N-Methylpyrrolidin (NMP) vorgelegt und mit 6,19 g K₃PO₄ versetzt. Die Suspension wurde auf 150 °C erwärmt und ein Vakuum von 300 mbar angelegt. 3-Chlor-2-Fluor-propionsäureethylester (4,1 g, 27 mmol) wurden zudosiert und gleichzeitig Produkt abdestilliert. Man erhielt 2,7 g 2-Fluoracrylsäureethylester als farblose Flüssigkeit, die mit ca. 15% NMP verunreinigt war. Das überschüssige NMP konnte mittels Extraktion mit Wasser entfernt werden. Man erhielt 1,3 g Produkt als farblose Flüssigkeit. (Ausbeute 42 %)
1H-NMR (400 mHz, CDCl3): 5,14 (ddd; J = 48 Hz, 5 Hz, 4 Hz; CHF); 4,32 (qd, J = 7 Hz, 1 Hz; CH2), 3,95 (dd, J = 4 Hz, 1 Hz, CHCl); 3,90 (dd, J = 5 Hz, 3 Hz, CHCl); 1,34 (t, J = 7 Hz, CH3) ppm.

## Patentansprüche

1. Verfahren zur Herstellung von Verbindungen der Formel (I) 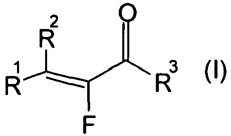
worin R¹ und R² gleich oder verschieden sind und unabhängig voneinander für Wasserstoff, C₁-C₁₅-Alkyl, C₆-C₂₄-Aryl, C₁-C₁₅-Alkoxy, C₂-C₁₀-Alkenyl, C₆-C₂₄-Aryloxy, C₇-C₁₅-Arylalkyl, C₁-C₁₅-Alkylthio, C₁-C₁₅-Mono- und Dialkylamino, C₆-C₂₄-Mono- und Diarylamino oder 5- bis 8- gliedriges gesättigtes oder ungesättigtes Heterozyklyl stehen, die weiter durch Reste ausgewählt aus der Gruppe C₁-C₁₅-Alkyl, C₂-C₁₀-Alkenyl, C₁-C₁₅-Alkoxy, C₆-C₂₄-Aryl, C₇-C₁₅-Arylalkyl, C₁-C₁₅-Alkylthio, Halogen, Hydroxy, Cyano, Nitro, Amino, Carboxy, C₁-C₁₆-Halogenalkyl, C₂-C₁₀-Halogenalkenyl, C₁-C₁₅-Halogenalkoxy, C₁-C₁₅-Halogenalkylthio und 5- bis 8-gliedriges gesättigtes oder ungesättigtes Heterozyklyl substituiert sein können, oder
R¹ und R² bilden zusammen einen carbozyklischen oder heterozyklischen, gesättigten oder ungesättigten 4- bis 8-gliedrigen Ring der weiter durch Reste ausgewählt aus der Gruppe C₁-C₁₅-Alkyl, C₂-C₁₀-Alkenyl, C₁-C₁₅-Alkoxy, C₆-C₂₄-Aryl, C₇-C₁₅-Arylalkyl, C₁-C₁₅-Alkylthio, Halogen, Hydroxy, Cyano, Nitro, Amino, Carboxy, C₁-C₁₅-Halogenalkyl, C₂-C₁₀-Halogenalkenyl, C₁-C₁₅-Halogenalkoxy, C₁-C₁₅-Halogenalkylthio und 5- bis 8- gliedriges gesättigtes oder ungesättigtes Heterozyklyl substituiert sein kann und
R³ steht für C₁-C₁₅-Alkoxy, C₁-C₁₅-Mono- und Dialkylamino, C₁-C₁₅-Alkylthio, C₆-C₂₄-Aryloxy, C₆-C₂₄-Mono- und Diarylamino und 5- bis 8- gliedriges gesättigtes oder ungesättigtes Heterozyklyl das über ein Heteroatom verknüpft ist, die weiter substituiert sein können durch Reste ausgewählt aus der Gruppe C₁-C₁₅-Alkyl, C₂-C₁₀-Alkenyl, C₁-C₁₅-Alkoxy, C₁-C₁₅-Alkylthio, C₆-C₂₄-Aryl, C₇-C₁₅-Arylalkyl, Halogen, Hydroxy, Cyano, Nitro, Amino, Carboxy, C₁-C₁₅-Halogenalkyl, C₂-C₁₀-Halogenalkenyl, C₁-C₁₅-Halogenalkoxy, C₁-C₁₅-Halogenalkylthio und 5- bis 8- gliedriges gesättigtes oder ungesättigtes Heterozyklyl,
bei dem Verbindungen der Formel (II) 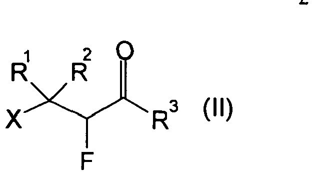
worin R¹, R² und R³ die oben genannte Bedeutung besitzen
und X für Cl, Br, I oder ein Pseudohalogen steht,
in Gegenwart mindestens einer Base ausgewählt aus der Gruppe Erdalkali- oder Alkalimetallhydroxide, -carbonate, -hydrogenphosphate und -phosphate
und in Gegenwart mindestens eines Polymerisationsinhibitors
zu Verbindungen der Formel (I) umgesetzt werden.

2. Verfahren nach Anspruch 1, **dadurch gekennzeichnet, dass** R¹ oder/und R² für Wasserstoff, C₁-C₆-Alkyl, C₆-C₂₄-Aryl, C₁-C₆-Alkoxy, C₂-C₆-Alkenyl, C₆-C₂₄-Aryloxy, C₇-C₁₀-Arylalkyl, C₁-C₆-Alkylthio, C₁-C₆-Mono- und Dialkylamino, C₆-C₂₄-Mono- und Diarylamino oder 5- bis 8- gliedriges gesättigtes oder ungesättigtes Heterozyklyl stehen.

3. Verfahren nach einem der Ansprüche 1 oder 2, **dadurch gekennzeichnet, dass** R³ für C₁-C₆-Alkoxy, C₁-C₈-Mono- und Dialkylamino, C₁-C₆-Alkylthio, C₆-C₂₄-Aryloxy, C₆-C₂₄-Mono- und Diarylamino oder 5- bis 8- gliedriges gesättigtes oder ungesättigtes Heterozyklyl das über ein Stickstoffatom verknüpft ist, steht.

4. Verfahren nach einem oder mehreren der Ansprüche 1 bis 3 **dadurch gekennzeichnet, dass** die Base Natriumcarbonat, Kaliumcarbonat, Natriumphosphat oder Kaliumphosphat oder Mischung dieser Basen ist.

5. Verfahren nach einem oder mehreren der Ansprüche 1 bis 4 **dadurch gekennzeichnet, dass** als hochsiedende, aprotische, polare Lösungsmittel Ethylenglycoldibutylether, Diethylenglykoldimethyether, Tetraethylenglycoldimethylether, Dimethylformamid, Dimethylsulfoxid, Sulfolan, ortho-Dichlorbenzol oder N-Methyl-pyrrolidon oder Mischungen solcher Lösungsmittel eingesetzt werden.

6. Verfahren nach einem oder mehreren der Ansprüche 1 bis 5, **dadurch gekennzeichnet, dass** als Polymerisationsinhibitoren 2,2,6,6-Tetramethylpiperidin-N-oxyl, Schwefel, p-Benzochinon, 4-tert.-Butylbrenzcatechin, Phenothiazin Di-tert-butylhydroxyltoluol (BHT) oder Mischungen dieser Polymerisationsinhibitoren eingesetzt werden.

7. Verfahren nach einem oder mehreren der Ansprüche 1 bis 6, **dadurch gekennzeichnet, dass** die Reaktion bei einer Temperatur von 130 °C bis 170 °C durchgeführt wird.

8. Verfahren nach einem oder mehreren der Ansprüche 1 bis 7, **dadurch gekennzeichnet, dass** die Stoffmengenverhältnisse der Verbindungen der Formel (II) und der eingesetzten Base zwischen 0,5 und 1,5 liegen.

9. Verfahren nach einem oder mehreren der Ansprüche 1 bis 8, **dadurch gekennzeichnet, dass** die Verbindung der Formel (I) während der Herstellung aus dem Reaktionsgemisch abgetrennt wird.

10. Verfahren nach einem oder mehreren der Ansprüche 1 bis 9, **dadurch gekennzeichnet**, das die Verbindungen der Formel (II) 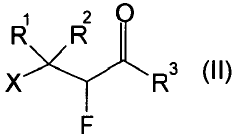
worin R¹,R² und R³ die unter Anspruch 1 aufgeführten Bedeutungen besitzen
und X für Cl, Br, I oder ein Pseudohalogen steht
durch Umsetzung der Verbindungen der Formel (III) 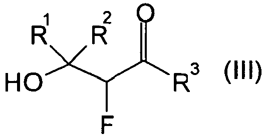 mit Halogenierungsmitteln hergestellt werden.

11. Verfahren nach Anspruch 10, **dadurch gekennzeichnet, dass** als Halogenierungsmittel Thionylchlorid, Thionylbromid, Phosphortrichlorid, Phosphortribromid, Sulfurylchlorid, Sulfurylbromid oder HX mit X = Cl, Br oder F oder Mischungen dieser Verbindungen eingesetzt werden.

12. Verfahren nach Anspruch 10, **dadurch gekennzeichnet, dass** das Verfahren in Gegenwart mindestens eines Lösungsmittels ausgewählt aus der Gruppe Benzol, Hexan, Petrolether, Toluol, p-Xylol und Xylol Isomerengemisch oder Mischungen solcher Lösungsmittel durchgeführt wird.

13. Verfahren nach einem der Ansprüche 10 bis 12, **dadurch gekennzeichnet, dass** das Verfahren in Gegenwart einer Base ausgewählt aus der Gruppe bi- oder trizyklischen Basen, Pyridin, N-Methylpiperidin, N,N-Dimethylaminopyridin oder Piperidin oder Mischungen dieser Basen durchgeführt wird.

14. Verfahren nach einem oder mehreren Ansprüchen 10 bis 13, **dadurch gekennzeichnet, dass** das Stoffmengenverhältnis von Verbindungen der Formel (III) zu den Halogenierungsmittel zwischen 1,0 und 1,5 liegt.

15. Verfahren nach einem oder mehreren der Ansprüche 10 bis 14, **dadurch gekennzeichnet, dass** das Gemisch aus Base und Lösungsmittel vor oder während der Zugabe der Verbindungen der Formel (III) und/oder der Halogenierungsmittel auf 60°C bis 90°C erwärmt wird.

## Claims

1. Process for the preparation of compounds of the formula (I) : 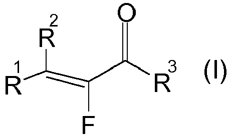
in which R¹ and R² are identical or different and are, independently of one another, hydrogen, C₁-C₁₅-alkyl, C₆-C₂₄-aryl, C₁-C₁₅-alkoxy, C₂-C₁₀-alkenyl, C₆-C₂₄-aryloxy, C₇-C₁₅-arylalkyl, C₁-C₁₅-alkylthio, C₁-C₁₅-mono- and dialkylamino, C₆-C₂₄-mono- and diarylamino or 5- to 8-membered saturated or unsaturated heterocyclyl, which in addition can be substituted by radicals chosen from the group consisting of C₁-C₁₅-alkyl, C₂-C₁₀-alkenyl, C₁-C₁₅-alkoxy, C₆-C₂₄-aryl, C₇-C₁₅-arylalkyl, C₁-C₁₅-alkylthio, halo, hydroxyl, cyano, nitro, amino, carboxyl, C₁-C₁₅-haloalkyl, C₂-C₁₀-haloalkenyl, C₁-C₁₅-haloalkoxy, C₁-C₁₅-haloalkylthio and 5- to 8-membered saturated or unsaturated heterocyclyl, or
R¹ and R² together form a carbocyclic or heterocyclic and saturated or unsaturated 4- to 8-membered ring which in addition can be substituted by radicals chosen from the group consisting of C₁-C₁₅-alkyl, C₂-C₁₀-alkenyl, C₁-C₁₅-alkoxy, C₆-C₂₄-aryl, C₇-C₁₅-arylalkyl, C₁-C₁₅-alkylthio, halo, hydroxyl, cyano, nitro, amino, carboxyl, C₁-C₁₅-haloalkyl, C₂-C₁₀-haloalkenyl, C₁-C₁₅-haloalkoxy, C₁-C₁₅-haloalkylthio and 5- to 8-membered saturated or unsaturated heterocyclyl, and
R³ is C₁-C₁₅-alkoxy, C₁-C₁₅-mono- and dialkylamino, C₁-C₁₅-alkylthio, C₆-C₂₄-aryloxy, C₆-C₂₄-mono- and diarylamino and 5- to 8-membered saturated or unsaturated heterocyclyl which is connected via a heteroatom, which in addition can be substituted by radicals chosen from the group consisting of C₁-C₁₅-alkyl, C₂-C₁₀-alkenyl, C₁-C₁₅-alkoxy, C₁-C₁₅-alkylthio, C₆-C₂₄-aryl, C₇-C₁₅-arylalkyl, halo, hydroxyl, cyano, nitro, amino, carboxyl, C₁-C₁₅-haloalkyl, C₂-C₁₀-haloalkenyl, C₁-C₁₅-haloalkoxy, C₁-C₁₅-haloalkylthio and 5- to 8-membered saturated or unsaturated heterocyclyl,
in which compounds of the formula (II): 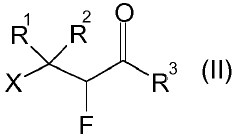
in which R¹, R² and R³ have the abovementioned meanings and X is Cl, Br, I or a pseudohalogen,
are reacted
in the presence of at least one base selected from the group of alkaline earth or alkali metal hydroxides, carbonates, hydrogenphosphates and phosphates
and in the presence of at least one polymerization inhibitor
to give compounds of the formula (I).

2. Process according to Claim 1, **characterized in that** R¹ and/or R² is hydrogen, C₁-C₆-alkyl, C₆-C₂₄-aryl, C₁-C₆-alkoxy, C₂-C₆-alkenyl, C₆-C₂₄-aryloxy, C₇-C₁₀-arylalkyl, C₁-C₆-alkylthio, C₁-C₆-mono- and dialkylamino, C₆-C₂₄-mono- and diarylamino or 5- to 8-membered saturated or unsaturated heterocyclyl.

3. Process according to either of Claims 1 and 2, **characterized in that** R³ is C₁-C₆-alkoxy, C₁-C₈-mono- and dialkylamino, C₁-C₆-alkylthio, C₆-C₂₄-aryloxy, C₆-C₂₄-mono- and diarylamino or 5- to 8-membered saturated or unsaturated heterocyclyl which is connected via a nitrogen atom.

4. Process according to one or more of Claims 1 to 3, **characterized in that** the base is sodium carbonate, potassium carbonate, sodium phosphate or potassium phosphate, or mixture of these bases.

5. Process according to one or more of Claims 1 to 4, **characterized in that** use is made, as high-boiling-point polar aprotic solvent, of ethylene glycol dibutyl ether, diethylene glycol dimethyl ether, tetraethylene glycol dimethyl ether, dimethylformamide, dimethyl sulfoxide, sulfolane, ortho-dichlorobenzene or N-methylpyrrolidone, or mixtures of such solvents.

6. Process according to one or more of Claims 1 to 5, **characterized in that** use is made, as polymerization inhibitors, of 2,2,6,6-tetramethylpiperidine N-oxyl, sulfur, p-benzoquinone, 4-tert-butylcatechol, phenothiazine, di-tert-butylhydroxytoluene (BHT) or mixtures of these polymerization inhibitors.

7. Process according to one or more of Claims 1 to 6, **characterized in that** the reaction is carried out at a temperature of 130°C to 170°C.

8. Process according to one or more of Claims 1 to 7, **characterized in that** the mole ratios of the compounds of the formula (II) to the base used are between 0.5 and 1.5.

9. Process according to one or more of Claims 1 to 8, **characterized in that** the compound of the formula (I) is separated from the reaction mixture during the preparation.

10. Process according to one or more of Claims 1 to 9, **characterized in that** the compounds of the formula (II) : 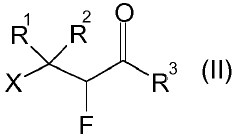
in which R¹, R² and R³ have the meanings listed in Claim 1
and X is Cl, Br, I or a pseudohalogen,
are prepared by reaction of the compounds of the formula (III): 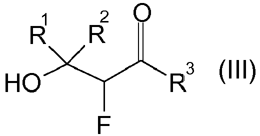 with halogenating agents.

11. Process according to claim 10, **characterized in that** use is made, as halogenating agent, of thionyl chloride, thionyl bromide, phosphorus trichloride, phosphorus tribromide, sulfuryl chloride, sulfuryl bromide or HX with X = Cl, Br or F, or mixtures of these compounds.

12. Process according to Claim 10, **characterized in that** the process is carried out in the presence of at least one solvent chosen from the group consisting of benzene, hexane, petroleum ethers, toluene, p-xylene and xylene isomer mixtures, or mixtures of such solvents.

13. Process according to one of Claims 10 to 12, **characterized in that** the process is carried out in the presence of a base chosen from the group consisting of bi- or tricyclic bases, pyridine, N-methylpiperidine, N,N-dimethylaminopyridine or piperidine, or mixtures of these bases.

14. Process according to one or more of Claims 10 to 13, **characterized in that** the mole ratio of compounds of the formula (III) to the halogenating agents is between 1.0 and 1.5.

15. Process according to one or more of Claims 10 to 14, **characterized in that** the mixture of base and solvent is heated to from 60°C to 90°C before or during the addition of the compounds of the formula (III) and/or of the halogenating agents.

## Revendications

1. Procédé de fabrication de composés de formule (I) 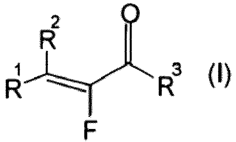
dans laquelle R¹ et R² sont identiques ou différents, et représentent indépendamment l'un de l'autre l'hydrogène, un alkyle en C₁-C₁₅, un aryle en C₆-C₂₄, un alcoxy en C₁-C₁₅, un alcényle en C₂-C₁₀, un aryloxy en C₆-C₂₄, un arylalkyle en C₇-C₁₅, un alkylthio en C₁-C₁₅, un mono- et dialkylamino en C₁-C₁₅, un mono- et diarylamino en C₆-C₂₄, ou un hétérocyclyle saturé ou insaturé de 5 à 8 chaînons, qui peuvent en outre être substitués par des radicaux choisis dans le groupe constitué par alkyle en C₁-C₁₅, alcényle en C₂-C₁₀, alcoxy en C₁-C₁₅, aryle en C₆-C₂₄, arylalkyle en C₇-C₁₅, alkylthio en C₁-C₁₅, halogène, hydroxy, cyano, nitro, amino, carboxy, halogénoalkyle en C₁-C₁₅, halogénoalcényle en C₂-C₁₀, halogénoalcoxy en C₁-C₁₅, halogénoalkylthio en C₁-C₁₅ et hétérocyclyle saturé ou insaturé de 5 à 8 chaînons, ou
R¹ et R² forment ensemble un cycle carbocyclique ou hétérocyclique saturé ou insaturé, de 4 à 8 chaînons, qui peut en outre être substitué par des radicaux choisis dans le groupe constitué par alkyle en C₁-C₁₅, alcényle en C₂-C₁₀, alcoxy en C₁-C₁₅, aryle en C₆-C₂₄, arylalkyle en C₇-C₁₅, alkylthio en C₁-C₁₅, halogène, hydroxy, cyano, nitro, amino, carboxy, halogénoalkyle en C₁-C₁₅, halogénoalcényle en C₂-C₁₀, halogénoalcoxy en C₁-C₁₅, halogénoalkylthio en C₁-C₁₅ et hétérocyclyle saturé ou insaturé de 5 à 8 chaînons, et
R³ représente un alcoxy en C₁-C₁₅, un mono- et dialkylamino en C₁-C₁₅, un alkylthio en C₁-C₁₅, un aryloxy en C₆-C₂₄, un mono- et diarylamino en C₆-C₂₄ et un hétérocyclyle saturé ou insaturé de 5 à 8 chaînons qui est relié par un hétéroatome, qui peuvent en outre être substitués par des radicaux choisis dans le groupe constitué par alkyle en C₁-C₁₅, alcényle en C₂-C₁₀, alcoxy en C₁-C₁₅, alkylthio en C₁-C₁₅, aryle en C₆-C₂₄, arylalkyle en C₇-C₁₅, halogène, hydroxy, cyano, nitro, amino, carboxy, halogénoalkyle en C₁-C₁₅, halogénoalcényle en C₂-C₁₀, halogénoalcoxy en C₁-C₁₅, halogénoalkylthio en C₁-C₁₅ et hétérocyclyle saturé ou insaturé de 5 à 8 chaînons,
selon lequel des composés de formule (II) 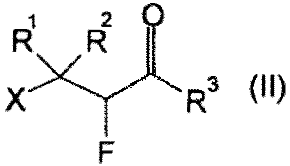
dans laquelle R¹, R² et R³ ont la signification indiquée précédemment,
et X représente Cl, Br, I ou un pseudohalogène,
sont mis en réaction en présence d'au moins une base choisie dans le groupe constitué par les hydroxydes, carbonates, hydrogénophosphates et phosphates de métaux alcalino-terreux ou alcalins,
et en présence d'au moins un inhibiteur de polymérisation,
pour former des composés de formule (I).

2. Procédé selon la revendication 1, **caractérisé en ce que** R¹ et/ou R² représentent l'hydrogène, un alkyle en C₁-C₆, un aryle en C₆-C₂₄, un alcoxy en C₁-C₆, un alcényle en C₂-C₆, un aryloxy en C₆-C₂₄, un arylalkyle en C₇-C₁₀, un alkylthio en C₁-C₆, un mono- et dialkylamino en C₁-C₆, un mono- et diarylamino en C₆-C₂₄, ou un hétérocyclyle saturé ou insaturé de 5 à 8 chaînons.

3. Procédé selon l'une quelconque des revendications 1 ou 2, **caractérisé en ce que** R³ représente un alcoxy en C₁-C₆, un mono- et dialkylamino en C₁-C₈, un alkylthio en C₁-C₆, un aryloxy en C₆-C₂₄, un mono- et diarylamino en C₆-C₂₄ ou un hétérocyclyle saturé ou insaturé de 5 à 8 chaînons qui est relié par un atome d'azote.

4. Procédé selon une ou plusieurs des revendications 1 à 3, **caractérisé en ce que** la base est le carbonate de sodium, le carbonate de potassium, le phosphate de sodium ou le phosphate de potassium ou un mélange de ces bases.

5. Procédé selon une ou plusieurs des revendications 1 à 4, **caractérisé en ce que** l'éther dibutylique d'éthylène glycol, l'éther diméthylique de diéthylène glycol, l'éther diméthylique de tétraéthylène glycol, le diméthylformamide, le diméthylsulfoxyde, le sulfolane, l'ortho-dichlorobenzène ou la N-méthyl-pyrrolidone ou des mélanges de tels solvants sont utilisés en tant que solvants polaires aprotiques de point d'ébullition élevé.

6. Procédé selon une ou plusieurs des revendications 1 à 5, **caractérisé en ce que** le 2,2,6,6-tétraméthylpipéridine-N-oxyle, le soufre, la p-benzoquinone, la 4-tert.-butylbrenzcatéchine, la phénothiazine, le di-tert-butylhydroxytoluène (BHT) ou des mélanges de ces inhibiteurs de polymérisation sont utilisés en tant qu'inhibiteurs de polymérisation.

7. Procédé selon une ou plusieurs des revendications 1 à 6, **caractérisé en ce que** la réaction est réalisée à une température de 130 °C à 170 °C.

8. Procédé selon une ou plusieurs des revendications 1 à 7, **caractérisé en ce que** les rapports entre les quantités de matière des composés de formule (II) et de la base utilisée se situent entre 0,5 et 1,5.

9. Procédé selon une ou plusieurs des revendications 1 à 8, **caractérisé en ce que** le composé de formule (I) est séparé du mélange réactionnel pendant la fabrication.

10. Procédé selon une ou plusieurs des revendications 1 à 9, **caractérisé en ce que** les composés de formule (II) 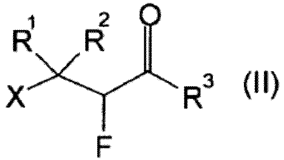
dans laquelle R¹, R² et R³ ont les significations indiquées dans la revendication 1,
et X représente Cl, Br, I ou un pseudohalogène,
sont fabriqués par mise en réaction des composés de formule (III) 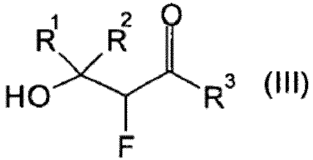 avec des agents d'halogénation.

11. Procédé selon la revendication 10, **caractérisé en ce que** le chlorure de thionyle, le bromure de thionyle, le trichlorure de phosphore, le tribromure de phosphore, le chlorure de sulfuryle, le bromure de sulfuryle ou HX avec X = Cl, Br ou F ou des mélanges de ces composés sont utilisés en tant qu'agents d'halogénation.

12. Procédé selon la revendication 10, **caractérisé en ce que** le procédé est réalisé en présence d'au moins un solvant choisi dans le groupe constitué par le benzène, l'hexane, l'éther de pétrole, le toluène, le p-xylène et un mélange d'isomères de xylène ou des mélanges de tels solvants.

13. Procédé selon l'une quelconque des revendications 10 à 12, **caractérisé en ce que** le procédé est réalisé en présence d'une base choisie dans le groupe constitué par les bases bi- ou tricycliques, la pyridine, la N-méthylpipéridine, la N,N-diméthylaminopyridine ou la pipéridine ou les mélanges de ces bases.

14. Procédé selon une ou plusieurs des revendications 10 à 13, **caractérisé en ce que** le rapport entre les quantités de matière des composés de formule (III) et des agents d'halogénation est compris entre 1,0 et 1,5.

15. Procédé selon une ou plusieurs des revendications 10 à 14, **caractérisé en ce que** le mélange d'une base et d'un solvant est porté à une température de 60 °C à 90 °C avant ou pendant l'ajout des composés de formule (III) et/ou des agents d'halogénation.
